# EUROPÄISCHE PATENTSCHRIFT

(11) **EP 1 463 453 B1**
(45) Veröffentlichungstag und Bekanntmachung des Hinweises auf die Patenterteilung: **22.02.2006**
(21) Anmeldenummer: 02806011.9
(22) Anmeldetag: 09.01.2002
(51) Int. Cl.: A61B 17/17

(54) **VORRICHTUNG ZUM BOHREN ODER EINBRINGEN VON IMPLANTATEN**
DEVICE FOR DRILLING OR FOR INSERTING IMPLANTS
DISPOSITIF POUR EFFECTUER UN PER AGE OU POSER DES IMPLANTS

(43) Veröffentlichungstag der Anmeldung: 06.10.2004
(73) Patentinhaber: Synthes AG Chur, 7002 Chur (CH)
(72) Erfinder: HÖNTZSCH, Dankward, 72076 Tübingen (DE); STEINER, Beatrice, CH-7265 Davos (CH); RUPP, Stephan, CH-7270 Davos (CH)
(74) Vertreter: Lusuardi, Werther
(86) Internationale Anmeldenummer: PCT/CH2002/000011
(87) Internationale Veröffentlichungsnummer: WO 2003/057051

(56) Entgegenhaltungen:
- WO-A-96/20650
- CH-A- 396 304
- JP-A- 9 149 907
- US-A- 5 445 641
- US-A- 6 033 430

## Beschreibung

Die Erfindung bezieht sich auf eine Vorrichtung für osteosynthetische Zwecke, insbesondere zum Bohren, Gewindeschneiden oder Einbringen von Implantaten in einen Knochen gemäss dem Oberbegriff des Patentanspruchs 1.

Eine Bohrbüchse, welche für osteosynthetische Zwecke anwendbar ist und insbesondere zum Bohren, Gewindeschneiden oder Einbringen von Implantaten dient, ist aus der WO 96120650 FRIGG bekannt. Diese bekannte Bohrbüchse umfasst eine Büchse, deren vorderes Ende an einem Knochen zur Anlage bringbar ist und durch deren hinteres Ende in Instrument oder Implantat einführbar ist. Ferner umfasst diese bekannte Bohrbüchse im Bereich des hinteren Endes einen Kühlmittelanschluss, durch welchen Kühlmittel in die Bohrbüchse einleitbar ist. Nachteilig an dieser bekannten Vorrichtung ist, dass beim Einbringen einer Knochenschraube diese in der Bohrung der Bohrbüchse nicht am Schraubenschaft geführt werden kann, da sonst die Bohrbüchse wegen des im Durchmesser grösseren Schraubenkopfes nicht mehr von der Knochenschraube entfembar ist.

Hier will die Erfindung Abhilfe schaffen. Der Erfindung liegt die Aufgabe zugrunde, eine Vorrichtung zu schaffen, mit welcher auch Schrauben deren Kopf einen grösseren Durchmesser aufweist als deren Schaft in den Hohlraum einführbar sind und die Vorrichtung nach dem Einschrauben der Schraube vom Implantat entfembar ist.

Die erfindungsgemässe Vorrichtung umfasst im wesentlichen eine auf ihrer gesamten, parallel zur Längsachse betrachteten Länge L getrennte Bohrbüchse mit einem Hohlraum und einem mit dem Hohlraum in Verbindung stehenden Kühlmittelanschluss. Durch die getrennte Ausführung der Bohrbüchse ist erreichbar, dass nach dem Einschrauben einer Schraube, welche durch den Hohlraum an das vordere Ende der Bohrbüchse gebracht wird, die Schalen der getrennten Bohrbüchse quer zur Längsachse relativ zueinander verschoben werden können und damit der Hohlraum der Bohrbüchse geöffnet wird und die Bohrbüchse über den im Durchmesser grösseren Schraubenkopf weggezogen werden kann.

In der bevorzugten Ausführungsform der erfindungsgemässen Vorrichtung verjüngt sich der Hohlraum gegen das vordere Ende der Bohrbüchse, so dass der Schraubenschaft einer in den Hohlraum eingebrachten Schraube durch die Hohlraumwand am vorderen Ende der Bohrbüchse geführt wird. Die Verjüngung des Hohlraumes kann beispielsweise konisch erfolgen.

Vorzugsweise ist die Bohrbüchse in zwei Halbschalen getrennt, deren Trennflächen in parallelen und zur Längsachse parallelen Ebenen liegen. In einer weiteren Ausführungsform der erfindungsgemässen Vorrichtung sind die Trennflächen beider Halbschalen abgestuft, wobei die Trennflächen der ersten Halbschale Erhebungen aufweisen und die Trennflächen der zweiten Halbschale dazu komplementäre Vertiefungen aufweisen. Durch die bei geschlossener Bohrbüchse ineinander eingreifenden axial angeordneten Erhebungen und Vertiefungen wird einerseits die Dichtheit der Bohrbüchse erhöht und andererseits die Stabilität gegen Verschiebungen der Halbschalen relativ zueinander und parallel zu den Trennflächen erhöht.

Ferner umfasst die erfindungsgemässe Vorrichtung in einer anderen Ausführungsform einen Haltegriff, welcher vorzugsweise gabelförmig mit zwei Stäben ausgebildet ist. Die beiden Stäbe sind an einem Ende des Haltegriffes miteinander verbunden, während am anderen Ende des Haltegriffes jeder Stab mit einer anderen Halbschale der Bohrbüchse verbunden ist. Durch diese Ausgestaltung des Haltegriffes können die Halbschalen relativ zueinander bewegt werden, so dass die Bohrbüchse wahlweise geöffnet oder geschlossen werden kann.

In wiederum einer anderen Ausführungsform der erfindungsgemässen Vorrichtung umfasst diese eine innere Bohrbüchse, welche in den Hohlraum einführbar ist. Die Zentralbohrung der inneren Bohrbüchse ist derart ausgestaltet, dass ein Bohrer zentriert und geführt werden kann, während anschliessend nach dem Entfernen der inneren Bohrbüchse Gewindeschneidwerkzeuge durch den Hohlraum geführt werden können.

In einer weiteren Ausführungsform der erfindungsgemässen Vorrichtung umfasst diese einen Trocar zur Zentrierung der Bohrbüchse in einer Plattenbohrung beispielsweise an einer Knochenplatte. Der Durchmesser des Trocars kann einerseits so ausgestaltet sein, dass der Trocar bei entfernter innerer Bohrbüchse durch die Hohlraumwand der Bohrbüchse geführt wird, oder andererseits so bemessen sein, dass der Trocar direkt in die Zentralbohrung der inneren Bohrbüchse einführbar ist. Durch die letztere Ausgestaltung des Trocars entfällt während der Implantation beispielsweise einer Knochenschraube das Auswechseln der Einsätze. Nach dem Zentrieren der Bohrbüchse mittels des Trocars wird dieser aus der Zentralbohrung der inneren Bohrbüchse herausgezogen und der Bohrer in die Zentralbohrung eingeführt. Nach abgeschlossenem Bohrvorgang wird die innere Bohrbüchse aus dem Hohlraum der Bohrbüchse entfernt und das Gewindeschneidwerkzeug in den Hohlraum der Bohrbüchse eingeführt.

Vorzugsweise ist das vordere Ende der Bohrbüchse aussen so ausgestaltet, dass sich die Bohrbüchse am vorderen Ende verjüngt. Diese Verjüngung kann beispielsweise konisch oder abgerundet ausgeführt sein und ermöglicht eine einfachere Zentrierung des vorderen Endes der Bohrbüchse in einer Bohrung oder Ansenkung für Schraubenköpfe beispielsweise an einer Knochenplatte.

Die Erfindung löst die gestellte Aufgabe mit einer Vorrichtung für osteosynthetische Zwecke, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung:
- während des chirurgischen Eingriffes- die Bohrbüchse beispielsweise in einem Schraubenloch einer Knochenplatte zentrierbar ist und dadurch ein Verrutschen der Bohrbüchse während des Eingriffes verhindert wird;
- ein Trocar in den Hohlraum der Bohrbüchse einführbar ist;
- durch die Bohrbüchse eine Längenmessung vorgenommen werden kann;
- die innere Bohrbüchse oder der Trocar stehen am vorderen Ende der Bohrbüchse vor, so dass ein Plattenloch ertastet werden kann; und
- eine Schraube ist bis zum Öffnen der beiden Halbschalen der Bohrbüchse im Hohlraum gefangen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Seitenansicht der bevorzugten Ausführungsform der erfindungsgemässen Vorrichtung;
Fig. 2 eine Aufsicht auf die in Fig. 1 dargestellte Ausführungsform der erfindungsgemässen Vorrichtung;
Fig. 3 einen Schnitt durch eine Ausführungsform der erfindungsgemässen Vorrichtung zusammen mit einer inneren Bohrbüchse;
Fig. 4 einen Schnitt durch eine Ausführungsform der erfindungsgemässen Vorrichtung zusammen mit einem Trocar,
Fig. 5 eine Schnitt durch eine weitere Ausführungsform der erfindungsgemässen Vorrichtung mit einer inneren Bohrbüchse und einem darin eingeführten Trocar, und
Fig. 6 einen Schnitt durch eine weitere Ausführungsform der erfindungsgemässen Vorrichtung mit einem Einsatz am vorderen Ende.

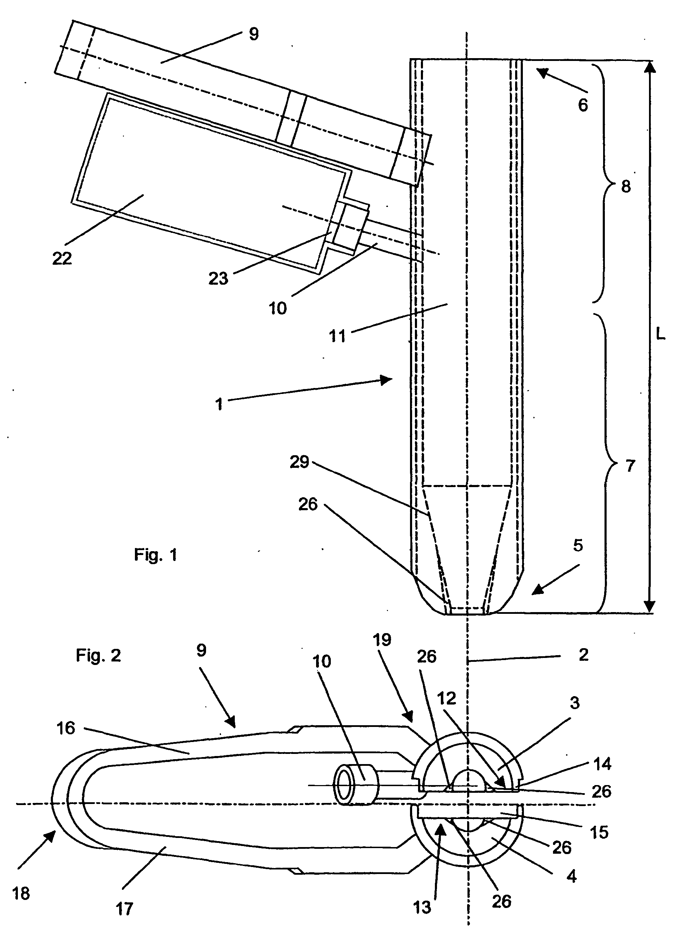

Fig. 1 und 2 zeigen die efindungsgemässe Vorrichtung als auf ihrer ganzen Länge L getrennte Bohrbüchse 1 mit zwei Halbschalen 3;4, deren parallel zur Längsachse 2 verlaufende Trennflächen 12;13 ineinander fügbar sind. Vom hinteren Ende 6 der Bohrbüchse 1 ist ein Instrument oder Implantat koaxial zur Längsachse 2 in den Hohlraum 11 einführbar. Der Hohlraum 11 ist auch am vorderen Ende 5 der Bohrbüchse 1 offen, so dass bei an einen Knochen oder ein Implantat angelegtem vorderen Ende 5 die Bohrbüchse 1 zur Führung eines im Hohlraum 11 axial verschiebbaren Bohrers oder anderen Instrumentes dient. An dem an das hintere Ende 6 der Bohrbüchse 1 angrenzenden, hinteren Segment 8 ist ein Haltegriff 9 und ein Kühlmittelanschluss 10 angebracht. Ein Kühlmittel ist durch den Kühlmittelanschluss 10 direkt in den Hohlraum 11 in der Bohrbüchse 1 einleitbar, wodurch beispielsweise ein Bohrer kühlbar ist. Die Trennflächen 12 der ersten Halbschale 3 weisen parallel zur Längsachse 2 je eine Erhebung 14, welche in die an den Trennflächen 13 der zweiten Halbschale 4 angebrachten komplementären Vertiefungen 15 einfügbar sind. Der gabelförmige Haltegriff 9 umfasst zwei Stäbe 16;17, welche am ersten Ende 18 des Haltegriffs 9 miteinander verbunden sind und am zweiten Ende 19 des Haltegriffs 9 je mit einer Halbschale 3;4 verbunden sind. Durch Auseinanderschieben der Stäbe 16;17 werden die Halbschalen 3;4 voneinander entfernt und die Bohrbüchse 1 wird parallel zur Längsachse 2 getrennt, während beim Zusammenschieben der Stäbe 16;17 die Halbschalen 3;4 an ihren Trennflächen 12;13 zusammenfügbar sind und somit die Bohrbüchse 1 geschlossen wird. Das vordere Ende 5 der Bohrbüchse 1 ist in der hier dargestellten Ausführungsform so ausgebildet, dass dieses Ende 5 der Bohrbüchse 1 in eine Vertiefung an einem Implantat (nicht gezeichnet), welche zur Aufnahme des Schraubenkopfes einer Knochenschraube dient, passgenau einführbar ist, so dass eine Zentrierung der Bohrbüchse 1 durch die Vertiefung in einer Knochenplatte erreichbar ist. Der Hohlraum 11 verjüngt sich durch einen Konus 29 im vorderen Segment 7 der Bohrbüchse 1 gegen das vordere Ende 5 hin. Durch den sich gegen das vordere Ende 5 der Bohrbüchse 1 verjüngenden Hohlraum 11 und die konvexe Ausgestaltung des vorderen Endes 5 aussen an der Bohrbüchse 1 lässt sich erreichen, dass beim Eindrehen einer Knochenschraube die zusammengepressten Halbschalen 3;4 nicht zwischen der Vertiefung in der Platte und der Knochenschraube eingeklemmt werden. In der hier dargestellten Ausführungsform der erfindungsgemässen Vorrichtung sind am vorderen Ende 5 der Bohrbüchse 1 drei axial aneinander angrenzende, zur Längsachse 2 koaxiale Konusse angebracht, welche durch ihre verschiedenen Konuswinkel eine konvexe Ausgestaltung des vorderen Endes 5 der Bohrbüchse 1 ergeben. Diese Konusse eignen sich zur Zentrierung des vorderen Endes 5 der Bohrbüchse 1 in einer Vertiefung zur Aufnahme eine Schraubenkopfes an einer Knochenplatte. Werden beispielsweise bei der Bestimmung der Schraubenlänge die beiden Halbschalen 3;4 leicht getrennt, rutscht durch diese Ausgestaltung des vorderen Endes 5 der Bohrbüchse 1 dieses nicht aus der Vertiefung für den Schraubenkopf, so dass die Zentrierung erhalten bleibt.

Beim Bohren kann der Bohrer gekühlt werden. Dazu dient ein Kühlmittelbehälter 22, dessen Öffnung 23 an den Kühlmittelanschluss 10 an der Bohrbüchse 1 anschliessbar ist, zur Verfügung. Die über die Platte vorstehende Länge der Knochenschraube kann durch den Hohlraum 11 bestimmt werden. Zum vollständigen Eindrehen der Knochenschraube werden die Halbschalen 3;4 getrennt, so dass der Schraubenkopf am vorderen Ende 5 der Bohrbüchse 1 durch den verjüngten Hohlraum 11 passt. Solange die Schraube nicht durch das geöffnete vordere Ende 5 der Bohrbüchse 1 hindurch gedreht wurde, kann diese mit geschlossener Bohrbüchse 1 wieder herausgenommen werden, ohne dass die Schraube in den Weichteilen verloren gehen kann. Am vorderen Ende 5 der Bohrbüchse 1 sind im Hohlraum 11 der Bohrbüchse 1 zwei Nuten 26 angebracht. Diese Nuten 26 verlaufen parallel zu den Trennflächen 12;13 und senkrecht zur Längsachse 2 betrachtet parallel zur Längsachse 2 der Bohrbüchse 1 und werden durch die Trennflächen 12;13 symmetrisch in ihrer Länge geteilt. Senkrecht zu den Trennflächen 12;13 betrachtet verlaufen die Nuten 26 unter einem Winkel zur Längsachse 2, wobei dieser Winkel kleiner als der halbe Konuswinkel des Konus 29 ist, so dass die Tiefe der Nuten 29 vom vorderen Ende 5 gegen das hintere Ende 6 der Bohrbüchse 1 abnimmt und die Nuten 29 in den Konus 29 auslaufen. Da aufgrund dieser beiden Nuten 26 die Halbschalen 3;4 zur Bestimmung beispielsweise der Schraubenlänge nur wenig getrennt werden müssen, wird vermieden, dass das vordere Ende 5 der Bohrbüchse 1 aus der Vertiefung des Plattenloches herausrutscht und die Vertiefung anschliessend durch die Weichteile nicht mehr oder nur schwer gefunden werden könnte.

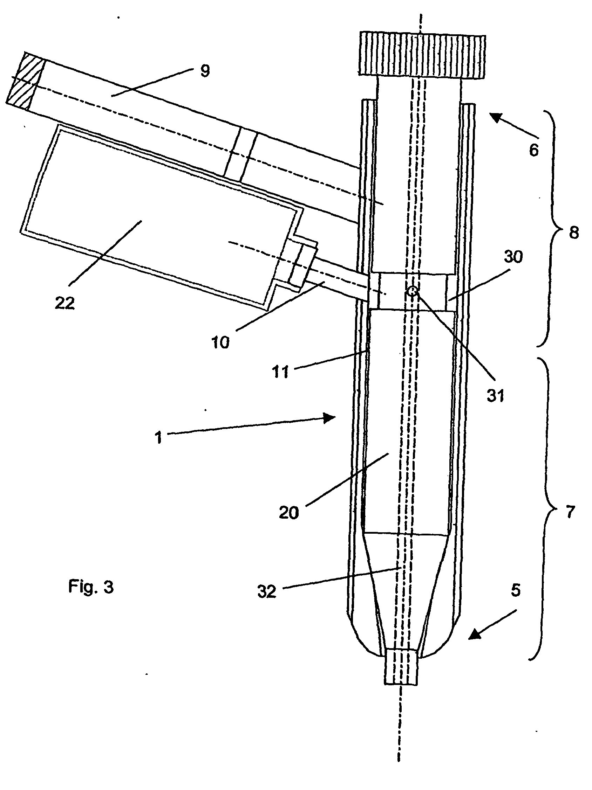

In Fig. 3 ist die erfindungsgemässe Vorrichtung mit einer Bohrbüchse 1 gemäss Fig. 1 und einer hohlzylindrischen inneren Bohrbüchse 20 dargestellt. Die innere Bohrbüchse 20 ist koaxial zur Längsachse 2 vom hinteren Ende 6 der Bohrbüchse 1 in den Hohlraum 11 einführbar und ragt im eingeführten Zustand über das vordere Ende 5 der Bohrbüchse 1 hinaus. Beim Bohrvorgang ist die innere Bohrbüchse 20 in die zweiteilige Bohrbüchse 1 eingesetzt. Während des Bohrens kann Kühlmittel aus dem Kühlmittelbehälter 22 in die Bohrbüchse 1 eingespritzt werden. Das Kühlmittel wird durch eine Ringnut 30 gepresst, welche auf der Höhe des Kühlmittelanschlusses 10 aussen an der inneren Bohrbüchse 20 angebracht ist, und tritt durch Bohrungen 31, welche bezüglich der Längsachse 2 radial in der Ringnut 30 angeordnet sind und in die Zentralbohrung 32 der inneren Bohrbüchse 20 reichen, in die Zentralbohrung 32 in der inneren Bohrbüchse 20 ein. Durch die Zentralbohrung 32 gelangt das Kühlmittel entlang des Bohrers zum vorderen Ende 5 der Bohrbüchse 1 und an die Spitze des Bohrers.

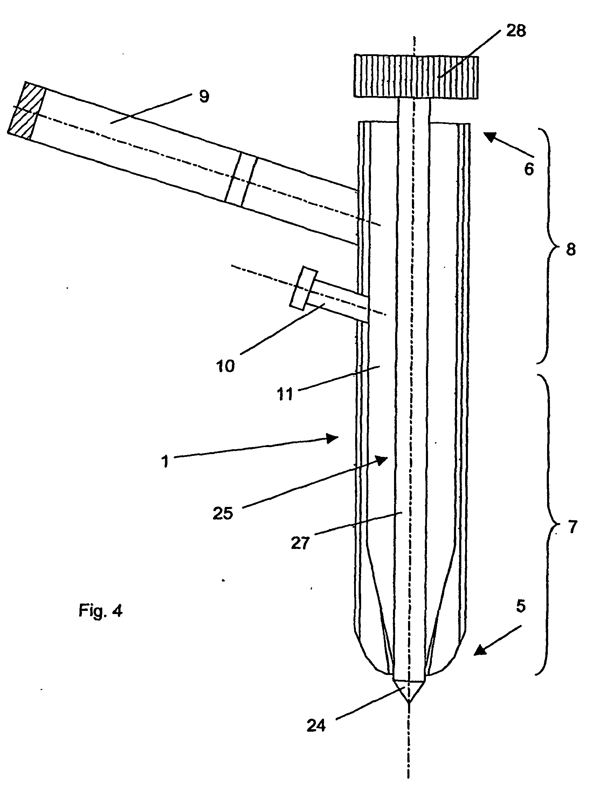

Fig. 4 zeigt die erfindungsgemässe Vorrichtung mit einer Bohrbüchse 1 wie in Fig. 1 dargestellt zusammen mit einem Trocar 25. Der Trocar 25 umfasst einen zylindrischen Schaft 27 mit einer Spitze 24 an seinem vorderen Ende und einen Handgriff 28 an seinem hinteren Ende. Zur Zentrierung der Bohrbüchse 1 wird zuerst der Trocar 25 vom hinteren Ende 6 der Bohrbüchse 1 in den Hohlraum 11 eingeführt bis die Spitze 24 des Trocars 25 über das vordere Ende 5 der Bohrbüchse 1 hinausragt.

Während des chirurgischen Eingriffes wird die Bohrbüchse 1 durch eine Stichinzision durch die Weichteile hindurch auf die untergeschobene Platte (nicht gezeichnet) geführt. Mit der über das vordere Ende 5 der Bohrbüchse 1 hinausragenden Spitze 24 der Trocars 25 können Bohrbüchse 1 und Trocar 25 gezielt zum Plattenloch geführt werden. Nachdem das Plattenloch gefunden ist, wird die Bohrbüchse 1 mit ihrem vorderen Ende 5 in die Vertiefung am Plattenloch eingeführt und wird von dieser zentriert. Bei der hier gezeigten Ausführungsform der Bohrbüchse 1 und des Trocars 25 wird nach dem Zentrieren der Bohrbüchse 1 in der Vertiefung am Plattenloch der Trocar 25 aus der Bohrbüchse 1 herausgenommen und die innere Bohrbüchse 20 eingeführt, so dass der Bohrer in die Zentralbohrung 32 der inneren Bohrbüchse 20 eingeführt werden kann und das Loch gebohrt werden kann. Anschliessend wird die innere Bohrbüchse 20 aus dem Hohlraum 11 in der Bohrbüchse 1 entfernt und das Gewinde durch den Hohlraum 11 in der Bohrbüchse 1 geschnitten.

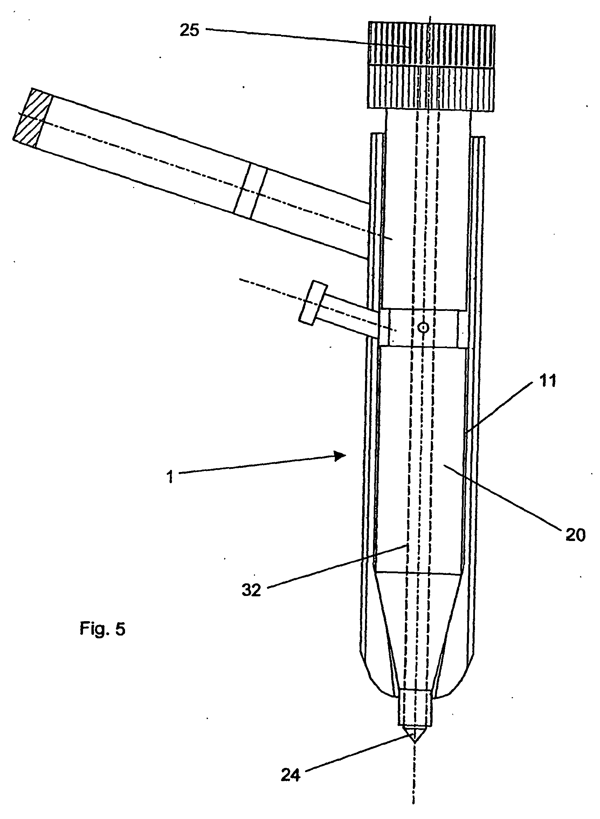

Eine andere Ausführungsform der erfindungsgemässen Vorrichtung ist in Fig. 5 dargestellt. Hier besteht die Möglichkeit, dass der Trocar 25 in die Zentralbohrung 32 der inneren Bohrbüchse 20 eingeführt wird, so dass die Spitze 24 des Trocars 25 am vorderen Ende 5 der Bohrbüchse 1 aus der Zentralbohrung 32 herausragt und über das vordere Ende 5 vorsteht. Damit ist erreichbar, dass nach dem Zentrieren der Vorrichtung lediglich der Trocar 25 aus der Zentralbohrung 32 der inneren Bohrbüchse 20 herausgezogen werden muss und dann der Bohrer in die Zentralbohrung 32 eingeführt werden kann und das Loch gebohrt werden kann. Anschliessend wird die innere Bohrbüchse 20 aus dem Hohlraum 11 der Bohrbüchse 1 entfernt und das Gewinde geschnitten.

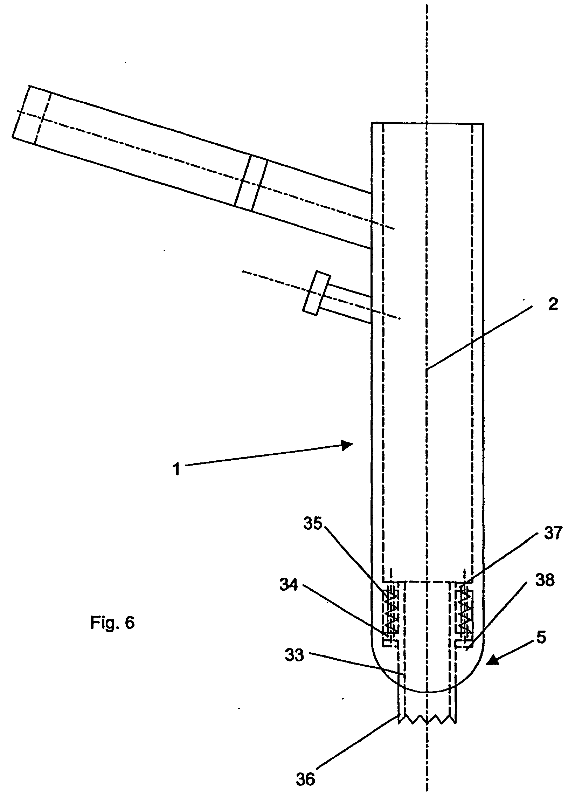

Eine weitere Ausführungsform ist in Fig. 6 dargestellt. Am vorderen Ende 5 der zweiteiligen Bohrbüchse 1 ist ein koaxial zur Längsachse 2 verschiebbarer Einsatz 33 angeordnet. Der Einsatz 33 ist hülsenförmig ausgestaltet und umfasst einen konzentrischen Bund 34. Die axiale Verschiebbarkeit des Einsatzes 33 wird dadurch begrenzt, dass der Bund 34 axial nur zwischen einem hinteren Anschlag 37 und einem vorderen Anschlag 38 in der Bohrbüchse 1 bewegbar ist. Zwischen dem hinteren Anschlag 37 und dem Bund 34 sind parallel zur Längsachse 2 Federn 35 angeordnet, mittels welcher der Einsatz 33 gegen das vordere Ende 5 der Bohrbüchse 1 gedrückt wird. Ferner ist das vordere Ende 36 des Einsatzes 33 gezackt ausgebildet.

## Patentansprüche

1. Vorrichtung für osteosynthetische Zwecke, insbesondere zum Bohren, Gewindeschneiden oder Einbringen von Implantaten in einen Knochen, mit
A) einer Bohrbüchse (1), welche eine Längsachse (2), dazu parallel eine Länge (L), ein hinteres Ende (6) zur Einführung eines Instrumentes oder Implantates, ein vorderes, auf ein Implantat oder einen Knochen aufsetzbares Ende (5) und koaxial zur Längsachse einen Hohlraum (11) umfasst; und
B) einem Kühlmittelanschluss (10, durch welchen ein Kühlmittel direkt in den Hohlraum (11) der Bohrbüchse (1) einleitbar ist.
**dadurch gekennzeichnet, dass**
C) die Bohrbüchse (1) zwei Halbschalen (3;4) umfasst, so dass sie auf ihrer gesamten Länge (L) trennbar ist; und
D) der Hohlraum (11) in der Bohrbüchse (1) sich gegen das vordere Ende (5) der Bohrbüchse (1) verjüngt.

2. Vorrichtung nach Anspruch 1, **dadurch gekennzeichnet, dass** die zwei Halbschalen (3;4) Trennflächen (12;13) aufweisen, welche parallel zur Längsachse (2) verlaufen.

3. Vorrichtung nach Anspruch 2, **dadurch gekennzeichnet, dass** die Trennflächen (12) der ersten Halbschale (3) Erhebungen (14) aufweisen und die Trennflächen (13) der zweiten Halbschale (4) zu den Erhebungen (14) komplementäre Vertiefungen (15) aufweisen.

4. Vorrichtung nach einem der Ansprüche 1 bis 3, **dadurch gekennzeichnet, dass** die Bohrbüchse (1) mit einem Haltegriff (9) ausgestattet ist.

5. Vorrichtung nach Anspruch 4, **dadurch gekennzeichnet, dass** der Haltegriff (9) gabelförmig mit zwei Stäben (16;17) ausgebildet ist und ein erstes Ende (18) und ein zweites Ende (19) umfasst, wobei die Stäbe (16;17) am ersten Ende (18) des Haltegriffs (9) miteinander verbindbar sind und am zweiten Ende (19) des Haltegriffs (9) jeder Stab (16;17) separat im Bereich des hinteren Endes (6) der Bohrbüchse (1) mit einer der Halbschalen (3;4) verbindbar ist.

6. Vorrichtung nach einem der Ansprüche 1 bis 5, **dadurch gekennzeichnet, dass** sie zusätzlich eine innere Bohrbüchse (20) umfasst, welche koaxial zur Längsachse (2) in den Hohlraum (11) einführbar ist.

7. Vorrichtung nach einem der Ansprüche 1 bis 6, **dadurch gekennzeichnet, dass** sie zusätzlich einen Trocar (25) umfasst.

8. Vorrichtung nach einem der Ansprüche 1 bis 7, **dadurch gekennzeichnet, dass** sie zusätzlich einen Kühlmittelbehälter (22) mit einer Öffnung (23) umfasst, wobei die Öffnung (23) an den Kühlmittelanschluss (10) anschliessbar ist, so dass Kühlmittel vom Kühlmittelbehälter (22) durch den Kühlmittelanschluss (10) in den Hohlraum (11) pressbar ist.

9. Vorrichtung nach einem der Ansprüche 1 bis 8, **dadurch gekennzeichnet, dass** sich das vordere Segment (7) der Bohrbüchse (1) gegen das vordere Ende (5) der Bohrbüchse (1) verjüngt.

## Claims

1. Device for osteosynthetic purposes, particularly for drilling, cutting threads or for inserting implants into a bone, comprising
A) a drill bush (1) having a longitudinal axis (2), parallel therewith a length (L), a rear end (6) for insertion of an instrument or an implant, a front end (5) for contacting an implant or a bone and a cavity (11) extending parallel to the longitudinal axis (11); and
B) a coolant supply (10) allowing a coolant to be directly fed into the cavity (11) of the drill bush (1),
**characterized in that**
C) the drill bush (1) comprises two semi-shells (3;4) such that it is separable on its entire length (L); and
D) the cavity (11) in the drill bush (1) tapers toward the front end (5) of the drill bush (1).

2. Device according to claim 1, **characterized in that** the two semi-shells (3;4) are provided with interfaces (12;13) extending parallel to the longitudinal axis (2)

3. Device according to claim 1 or 2, **characterized in that** the interfaces (12) of the first semi-shell (3) are provided with protrusions (14) and the interfaces (13) of the second semi-shell (4) are provided with recesses (15) being complementary to the protrusions (14).

4. Device according to one of the claims 1 to 3, **characterized in that** the drill bush (1) is provided with a handle (9).

5. Device according to claim 4, **characterized in that** the handle (9) is configured forked with two rods (16;17) and comprises a first end (18) and a second end (19) whereby the rods (16;17) are connectable with each other at the first end (18) of the handle (9) and whereby the rods (16;17) are connectable with one of the semi-shells (3;4) each in the range of the rear end (6) of the drill bush (1).

6. Device according to one of the claims 1 to 5, **characterized in that** it additionally comprises an inner drill bush (20) being insertable into the cavity (11) coaxially to the longitudinal axis (2).

7. Device according to one of the claims 1 to 6, **characterized in that** it additionally comprises a trocar (25).

8. Device according to one of the claims 1 to 7, **characterized in that** it additionally comprises a coolant container (22) with an opening (23), whereby the opening (23) is connectable to the coolant supply (10) such that a coolant is pressable from the coolant container (22) into the cavity (11) through the coolant supply (10).

9. Device according to one of the claims 1 to 8, **characterized in that** the front portion (7) of the drill bush (1) tapers toward the front end (5) of the drill bush (1).

## Revendications

1. Dispositif destiné à des fins d'ostéosynthèse, notamment au perçage, au filetage ou à la pose d'implants dans un os, comportant
A) une bague de perçage (1) qui comprend un axe longitudinal (2) et, parallèlement à celui-ci, une longueur (L), une extrémité arrière (6) permettant d'insérer un instrument ou un implant, une extrémité avant (5) pouvant être appliquée sur un implant ou un os et, coaxialement audit axe longitudinal, un espace vide (11); et
B) un raccord d'amenée de réfrigérant (10) par l'intermédiaire duquel un réfrigérant peut être introduit directement dans l'espace vide (11) de la bague de perçage (1);
**caractérisé en ce que**
C) la bague de perçage (1) comprend deux demi-coquilles (3;4), de telle sorte qu'elle peut être séparée sur l'ensemble de sa longueur (L); et
D) l'espace vide (11) ménagé dans la bague de perçage (1) va en se rétrécissant vers l'extrémité avant (5) de la bague de perçage (1).

2. Dispositif selon la revendication 1, **caractérisé en ce que** les deux demi-coquilles (3;4) présentent des surfaces de séparation (12;13) qui s'étendent parallèlement à l'axe longitudinal (2).

3. Dispositif selon la revendication 2, **caractérisé en ce que** les surfaces de séparation (12) de la première demi-coquille (3) présentent des saillies (14) et que les surfaces de séparation (13) de la deuxième demi-coquille (4) présentent des creux (15) complémentaires aux saillies (14).

4. Dispositif selon l'une des revendications 1 à 3, **caractérisé en ce que** la bague de perçage (1) est équipée d'une poignée de maintien (9).

5. Dispositif selon la revendication 4, **caractérisé en ce que** la poignée de maintien (9) est réalisée en forme de fourche comportant deux tiges (16;17) et comprend une première extrémité (18) et une deuxième extrémité (19), les tiges (16;17) pouvant être reliées l'une à l'autre au niveau de la première extrémité (18) de la poignée de maintien (9) et chaque tige (16;17) pouvant, au niveau de la deuxième extrémité (19) de la poignée de maintien (9), être reliées séparément, dans la zone de l'extrémité arrière (6) de la bague de perçage (1), à l'une des demi-coquilles (3;4).

6. Dispositif selon l'une des revendications 1 à 5, **caractérisé en ce qu'**il comprend en outre une bague de perçage intérieure (20) qui peut être insérée, coaxialement à l'axe longitudinal (2), dans l'espace vide (11).

7. Dispositif selon l'une des revendications 1 à 6, **caractérisé en ce qu'**il comprend en outre un trocart (25).

8. Dispositif selon l'une des revendications 1 à 7, **caractérisé en ce qu'**il comprend en outre un réservoir de réfrigérant (22) pourvu d'une ouverture (23), l'ouverture (23) pouvant être branchée à un raccord d'amenée de réfrigérant (10), de sorte que du réfrigérant provenant du réservoir de réfrigérant (22) peut être pressé, par l'intermédiaire du raccord d'amenée de réfrigérant (10), dans l'espace vide (11).

9. Dispositif selon l'une des revendications 1 à 8, **caractérisé en ce que** le segment avant (7) de la bague de perçage (1) va en se rétrécissant vers l'extrémité avant (5) de la bague de perçage (1).
